# EUROPEAN PATENT SPECIFICATION

(11) **EP 4 107 141 B1**
(45) Date of publication and mention of the grant of the patent: **03.04.2024**
(21) Application number: 21706178.7
(22) Date of filing: 08.02.2021
(51) Int. Cl.: C07C 29/00, C07C 29/132, C07C 29/141, C07C 29/60

(54) **PROCESS FOR PREPARING ALKYLENE GLYCOL MIXTURE FROM A CARBOHYDRATE SOURCE WITH DECREASED SELECTIVITY FOR POLYOL SIDE PRODUCTS**
VERFAHREN ZUR HERSTELLUNG EINES ALKYLENGLYKOLGEMISCHES AUS EINER KOHLENHYDRATQUELLE MIT VERMINDERTER SELEKTIVITÄT FÜR POLYOLNEBENPRODUKTE
PROCÉDÉ DE PRÉPARATION DE MÉLANGE D'ALKYLÈNE GLYCOL À PARTIR D'UNE SOURCE D'HYDRATES DE CARBONE AYANT UNE SÉLECTIVITÉ RÉDUITE POUR LES PRODUITS SECONDAIRES À BASE DE POLYOLS

(30) Priority: 17.02.2020 EP 20157690
(43) Date of publication of application: 28.12.2022
(73) Proprietor: Avantium Knowledge Centre B.V., 1014 BV Amsterdam (NL)
(72) Inventor: ANSOVINI, Davide, 1014 BV Amsterdam (NL); CLAASSENS-DEKKER, Paula, 1014 BV Amsterdam (NL); SINGH, Jagdeep, 1014 BV Amsterdam (NL)
(74) Representative: Avantium Intellectual Property
(86) International application number: PCT/EP2021/052949
(87) International publication number: WO 2021/165082

(56) References cited:
- US-A1- 2011 046 419

## Description

### Introduction

The present invention relates to a process for preparing a mixture of alkylene glycols (e.g. ethylene glycol and/or propylene glycol) from a carbohydrate source by catalytic conversion with hydrogen. More specifically, the catalytic hydrogenolysis process of the invention has a decreased selectivity for larger polyols like sorbitol and erythritol, which larger polyols are obtained generally as a side product in catalytic hydrogenolysis, when viewed in comparison to the selectivity for small alkylene glycols (like ethylene glycol and propylene glycol).

### Background of the invention

Alkylene glycols such as ethylene glycol and propylene glycol are valuable products or intermediates in chemical industry, as such compounds are used in various chemical processes. Traditionally, alkylene glycols are produced from fossile sources. More recently, there is ongoing research to produce alkylene glycols from renewable sources.

In this connection, CN 102643165 describes a process for producing ethylene glycol and propylene glycol from soluble sugars or starch. Similarly, US 7960594 discloses a process in which ethylene glycol is produced from cellulose. In WO 2016/114661 it is stated that ethylene glycol may be obtained from a carbohydrate source by catalytic reaction with hydrogen, which carbohydrate source may be obtained from a variety of sources, such as polysaccharides, oligosaccharides, disaccharides and monosaccharides (which all may be obtained from renewable sources). Suitable examples are stated to be cellulose, hemicellulose, starch, sugars such as sucrose, mannose, arabinose, glucose and mixtures thereof. Only glucose is exemplified as a starting material.

Most references such as US2011/0312488 and WO2017/097839 disclosing processes to obtain alkylene glycols like ethylene glycol or propylene glycol from (renewable) carbohydrates refer in a general sense to whole ranges of carbohydrates as potential starting material, but exemplify often only glucose, starch or cellulose as carbohydrate source.

The processes in the above references and others generally produce ethylene glycol in a selectivity up to 50 to 60%, based on the carbohydrate feed, when glucose is used as a feed, next to a whole range of other components in varying amounts. Next to ethylene glycol, smaller amounts of propylene glycol are obtained in such reactions. Apart from these two desired products, lower alkanols, butanediol (both 1,2-and 1,4-), and polyols like glycerol, erythritol, and sorbitol are formed as side products in the product mix.

US 2011/0046419 discloses a process for producing ethylene glycol in a sealed high-pressure reactor with a catalyst system. The catalyst system includes a first and a second active ingredient. The first active ingredient includes a transition metal of Group 8, 9 or 10 selected from iron, cobalt, nickel, ruthenium, rhodium, palladium, iridium and platinum. The second active ingredient includes a metallic state of molybdenum and/or tungsten, or a carbide, nitride, or phosphide thereof.

The world market for ethylene glycol is huge, the market for propylene glycol is smaller but still attractive. After removal of volatiles like lower alkanols, both ethylene glycol and propylene glycol can be obtained in pure form from the reaction mixture easily, as these components can be distilled off without too much problems. Also glycerol and butanediols can be removed, e.g. by distillation. This still leaves erythritol and sorbitol as bottom products, which are also more difficult to separate from eachother. There is a market value for food grade sorbitol, but sorbitol which has been processed in a process with heavy metal catalyst is not so easy to obtain in a food-grade purity. To avoid having to purify a considerable amount of erythritol and sorbitol, it is desired that the amounts in which these products are obtained is preferably as low as possible, whilst obtaining considerable amounts of commercially attractive components which can be obtained easily, like ethylene glycol and propylene glycol. Preferably, such should not be at the expense of total conversion.

Hence, there is a need for a process for preparing alkylene glycols from carbohydrates by reaction with hydrogen, in which reaction there is a decreased selectivity for sorbitol and erythritol, such process still yielding economically attractive amounts of valuable smaller alkane glycols like ethylene glycol and propylene glycol.

### Summary of the invention

It has now been found that the above objective can be met, at least in part, by a process for producing a mixture of 2 to 40% by weight of glycols dissolved in water with a ratio of between 1:5 to 1 : 100 for the combined selectivity for erythritol and sorbitol : the combined selectivity for ethylene glycol and propylene glycol, of the reaction products produced by said process, which process comprises feeding to a pressurized, continuously stirred tank reactor hydrogen and an aqueous feed solution comprising water and a carbohydrate, wherein the reactor contains a catalyst system which comprises a homogeneous catalyst comprising a tungsten compound and a heterogeneous catalyst comprising a hydrogenolysis metal selected from the groups 8, 9 or 10 of the Periodic Table of the Elements, characterized in that the carbohydrate in the aqueous feed comprises at least 80% of sucrose, by weight based on the total amount of carbohydrate in the aqueous feed.

In other words, it was found that the amount of less desired components such as erythritol and sorbitol (which are produced by hydrogenolysis of carbohydrates using hydrogen and a catalyst system comprising a homogeneous catalyst and a heterogeneous catalyst) can be reduced if one ensures that the feed of carbohydrates comprise a substantial amount of sucrose (e.g. at least 80% on the weight of carbohydrates in the feed). Or put differently, the selectivity for erythritol and sorbitol can be decreased when compared to ethylene glycol and propylene glycol if the carbohydrate feed comprises a substantial amount of sucrose, rather than the more common glucose. The present invention is such that sucrose can directly (i.e. without the need for hydrolysis into the monosaccharides glucose and fructose) be fed to the reactor (in solution).

### Detailed description of the invention

Depending on the amount of sucrose in the feed and detailed reaction conditions, the selectivity can be pushed further towards preferred products ethylene glycol plus propylene glycol, at the expense of e.g. erythritol and/or sorbitol. Hence, in the present invention it is preferred that the combined selectivity for erythritol and sorbitol : the combined selectivity for ethylene glycol and propylene glycol of the reaction products produced by said process is at least 1 : 7, preferably at least 1 : 10, more preferably at least 1 : 15.

To this end, and also for reasons of easier handling of the feed, it is preferred that the carbohydrate in the aqueous feed comprises at least 90% of sucrose, preferably at least 95% by weight of sucrose, by weight based on the total amount of carbohydrate in the aqueous feed. Most preferably, the feed is only sucrose, but in industrial sucrose minor amounts (e.g. 1-5% by weight) of other carbohydrates can still be present, which are not detrimental to the outcome. The reaction is preferably a continuous process, and the aqueous feed solution comprising water and a carbohydrate in the present process preferably comprises between 5 and 35% by weight of carbohydrate, preferably between 10 and 30% by weight of carbohydrate (by weight on the total feed).

As stated, the carbohydrate source containing sucrose is converted into a product mix comprising ethylene glycol and propylene glycol with hydrogen and a catalyst system which comprises a homogeneous catalyst comprising a tungsten compound and a heterogeneous catalyst comprising a hydrogenolysis metal selected from the groups 8, 9 or 10 of the Periodic Table of the Elements. Concerning the latter, it is preferred that the hydrogenolysis metal from groups 8, 9 or 10 of the Periodic Table of the Elements in this connection is selected from the group consisting of Cu, Fe, Ni, Co, Pd, Pt, Ru, Rh, Ir, Os and combinations thereof. Ruthenium is the preferred metal selected from the groups 8, 9 or 10 of the Periodic Table of the Elements in the present invention. It is preferred in the present invention that the amount of the hydrogenolysis metal selected from the groups 8, 9 or 10 of the Periodic Table of the Elements which present in the reactor is preferably present in an amount of between 0.05 and 20 g hydrogenolysis metal / L of reactor volume, more preferably between 0.1 and 12 g g hydrogenolysis metal / L of reactor volume, and most preferably between 0.5 and 8 g hydrogenolysis metal / L of reactor volume.

The hydrogenolysis metal catalyst referred to above can be present as such, but it is preferred that such is present in the form of a catalyst supported on a carrier. Preferred carriers in this case are carriers selected from the group supports, consisting of activated carbon, silica, alumina, silica-alumina, zirconia, titania, niobia, iron oxide, tin oxide, zinc oxide, silica-zirconia, zeolites, aluminosilicates, titanosilicates, magnesia, silicon carbide, clays and combinations thereof. Activated carbon is a preferred carrier in the present invention, in particular with the hydrogenolysis catalyst being ruthenium.

Next to the metal selected from groups 8, 9 or 10 of the Periodic Table of the Elements (the heterogeneous catalyst part) the catalyst system comprises a homogeneous catalyst part, which is herein a tungsten compound. In the process according to the present invention, it is preferred that the homogeneous catalyst comprising a tungsten compound is selected from the group consisting of tungstic acid (H₂WO₄), ammonium tungstate, ammonium metatungstate, ammonium paratungstate, tungstate compounds comprising at least one Group 1 or 2 element, metatungstate compounds comprising at least one Group 1 or 2 element, paratungstate compounds comprising at least one Group 1 or 2 element, tungsten oxide (WO₃), heteropoly compounds of tungsten, and combinations thereof. A most preferred homogeneous catalyst in the present reaction comprises tungstic acid and/or a tungstate, e.g. ammonium tungstate, sodium tungstate or potassium tungstate. The homogeneous catalyst comprising a tungsten compound (preferably comprising tungstic acid) in the present invention is preferably dissolved or dispersed in water and/or an alkylene glycol, the latter preferably being ethylene glycol.

The presently claimed process is preferably carried out as a continuous process. To this end, to the reactor are continuously or periodically added: a stream comprising a carbohydrate feed, and the same for pressurized hydrogen gas. Preferably also the homogeneous catalyst comprising a tungsten compound is continuously or periodically added to the reactor. The amount of catalyst in the feed to the reactor is preferably such that the concentration of the homogeneous catalyst comprising a tungsten compound present in the reactor is between 0.05 and 5 wt.%, preferably between 0.1 and 2 wt.% (calculated as tungsten metal).

In the present invention the amount of the homogeneous catalyst comprising a tungsten compound and a heterogeneous catalyst comprising a hydrogenolysis metal selected from the groups 8, 9 or 10 of the Periodic Table of the Elements are present in the reactor in amounts such that the weight ratio of weight of tungsten to the total weight of hydrogenolysis metal, all calculated on metal basis, is between 1: 3000 to 50 : 1 (tungsten metal : transition metal wt : wt).

The process of the present invention is carried out at elevated pressure (i.e. higher than atmospheric). Preferably, the total pressure in the reactor is between 2.0 and 16 MPa, preferably between 4 and 12 MPa, most preferably between 5 and 10 MPa. As hydrogen is key to the present reaction, pressurization is preferably carried out with hydrogen. Furthermore, it is preferred in the present invention that the aqueous feed solution comprising water and a carbohydrate comprises between 40% and 85% of water, between 5 and 35% by weight of carbohydrate, and between 5 to 40% of an alkylene glycol co-solvent (preferably ethylene glycol), all by weight on the total aqueous feed solution.

The reaction is preferably carried out such that the temperature in the reactor is between 150 and 270°C, preferably between 180 and 250°C. The rate of addition of aqueous feed solution comprising water and a carbohydrate into the CSTR is such that WHSV is preferably between 0.01 and 100 hr⁻¹, preferably between 0.05 and 10 hr⁻¹, more preferably between 0.5 and 2 hr⁻¹.

The invention further relates to a process for producing glycols dissolved in water, which process comprises feeding to a pressurized, continuously stirred tank reactor hydrogen an aqueous feed solution comprising water and a carbohydrate, wherein the reactor comprises a catalyst system which comprises a homogeneous catalyst comprising a tungstic acid and a heterogeneous catalyst comprising ruthenium supported on a carrier, characterized in that the carbohydrate in the aqueous feed comprises at least 80% of sucrose, by weight based on the total amount of carbohydrate in the aqueous feed, as it was found that the above objective can be met, at least in part, by this process.

Preferred carriers in this case for the ruthenium are carriers selected from the group supports, consisting of activated carbon, silica, alumina, silica-alumina, zirconia, titania, niobia, iron oxide, tin oxide, zinc oxide, silica-zirconia, zeolites, aluminosilicates, titanosilicates, magnesia, silicon carbide, clays and combinations thereof. Activated carbon is a preferred carrier in the present invention.

The presently claimed process is preferably carried out as a continuous process. To this end, to the reactor are continuously or periodically added: a stream comprising a carbohydrate feed, and the same for pressurized hydrogen gas. Preferably also the homogeneous catalyst comprising a tungsten compound is continuously or periodically added to the reactor. The amount of catalyst in the feed to the reactor is preferably such that the concentration of the homogeneous catalyst comprising tungstic acid present in the reactor is between 0.05 and 5 wt.%, preferably between 0.1 and 2 wt.% calculated as tungsten metal.

It is preferred in the present invention that the amount of ruthenium which present in the reactor is preferably present in an amount of between 0.05 and 20 g ruthenium / L of reactor volume, more preferably between 0.1 and 12 g ruthenium / L of reactor volume, and most preferably between 0.5 and 8 g ruthenium / L of reactor volume. The amount of the homogeneous catalyst comprising tungstic acid and a heterogeneous catalyst comprising ruthenium are present in the reactor in amounts such that the weight ratio of weight of tungsten to the total weight of hydrogenolysis metal, all calculated on metal basis, is between 1: 3000 to 50 : 1 (tungsten metal : transition metal wt : wt).

In the above process, the homogeneous catalyst comprising a tungstic acid is preferably dissolved or dispersed in water and/or an alkylene glycol, the latter preferably being ethylene glycol.

In this process, the carbohydrate in the aqueous feed preferably comprises at least 90% of sucrose, more preferably at least 95% by weight of sucrose, by weight based on the total amount of carbohydrate in the aqueous feed. Furthermore, it is preferred that in the now claimed process the aqueous feed solution comprising water and a carbohydrate preferably comprises between 5 and 35% by weight of carbohydrate, preferably between 10 and 30% by weight of carbohydrate.

The process of the present invention is carried out at elevated pressure (i.e. higher than atmospheric). Preferably, the total pressure in the reactor is between 2.0 and 16 MPa, preferably between 4 and 12 MPa, most preferably between 5 and 10 MPa. As hydrogen is key to the present reaction, pressurization is preferably carried out with hydrogen. Furthermore, it is preferred in the present invention that the aqueous feed solution comprising water and a carbohydrate comprises between 40% and 85% of water, between 5 and 35% by weight of carbohydrate, and between 5 to 40% of an alkylene glycol co-solvent (preferably ethylene glycol), all by weight on the total aqueous feed solution.

The reaction is preferably carried out such that the temperature in the reactor is between 150 and 270°C, preferably between 180 and 250°C. The rate of addition of aqueous feed solution comprising water and a carbohydrate into the CSTR is such that WHSV is preferably between 0.01 and 100 hr⁻¹, preferably between 0.05 and 10 hr⁻¹, more preferably between 0.5 and 2 hr⁻¹.

### Examples

Example 1: sucrose (purity > 99%) as feed carbohydrate in hydrogenolysis
Comparative 1: glucose (purity > 99%) as feed carbohydrate in hydrogenolysis

### Process description

Hydrogenolysis experiments were carried out in a continuously stirred tank reactor. The amount of liquid in the reactor was about 170 ml. Trials were done with two different residence times:
about 24 minutes residence time (example 1a and comparative 1a) and
about 34 minutes residence time (example 1b and comparative 1b).

The reactor contained as heterogeneous catalyst ruthenium on activated carbon. The amount of ruthenium on activated carbon was about 5 wt% Ru on AC. The total weight of heterogeneous catalyst on carrier was about 7 g Ru + AC for example 1a and comparative 1a, and about 4.3 g for Ru + AC for example 1b and comparative 1b. The reactor was filled with Ru/AC and water before the reactor was heated and pressurized. All of the heterogeneous catalyst remained in the reactor during the reaction.

The carbohydrate feed was prepared by dissolving the sucrose (examples 1a and 1b) and glucose (comparatives 1a and 1b) in a mixture of water and ethylene glycol at a concentration of about 20 wt% on the final feed composition which further contained about 60 wt% water and 20 wt% ethylene glycol.

The homogeneous catalyst solution was prepared by dissolving sodium hydroxide and H₂WO₄ in ethylene glycol, at a molar ratio of 0.7 : 1, to arrive at a concentration H₂WO₄ of 0.44 wt %.

The carbohydrate feed solution and homogeneous catalyst solution were mixed prior to use.

The reactor was heated to 220°C and pressurised with hydrogen gas to 65 bar. Hydrogen gas was entered into the reactor at a flow of 2000 ml/minute.

At the start of the reaction (t = 0 minutes) the mixture of carbohydrate feed and homogeneous catalyst solution was pumped into the reactor at a steady flow to obtain the residence times indicated (flow rates added to the reactor at 5 or 7 ml per minute.

Reactions were carried out for about 300 minutes, and from the outlet stream samples were taken at 8 - 10 times in the interval from 0 to 300 minutes.

### Results

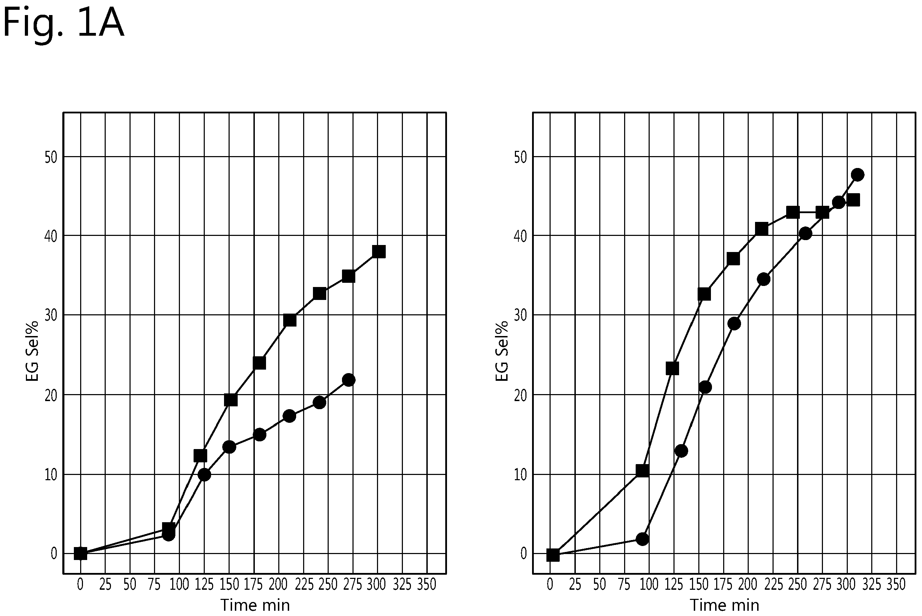

The samples obtained were analysed on concentration of polyol (ethylene glycol, propylene glycol, erythritol and sorbitol) using HPLC and from this reaction selectivities were calculated. The results are set out in figures 1A to 1D.
Figure 1A: selectivity of ethylene glycol obtained in the product stream, for sucrose as feed (squares) and glucose as feed (circles) for a residence time of about 24 minutes (left hand) and for a residence time of about 34 minutes (right hand).

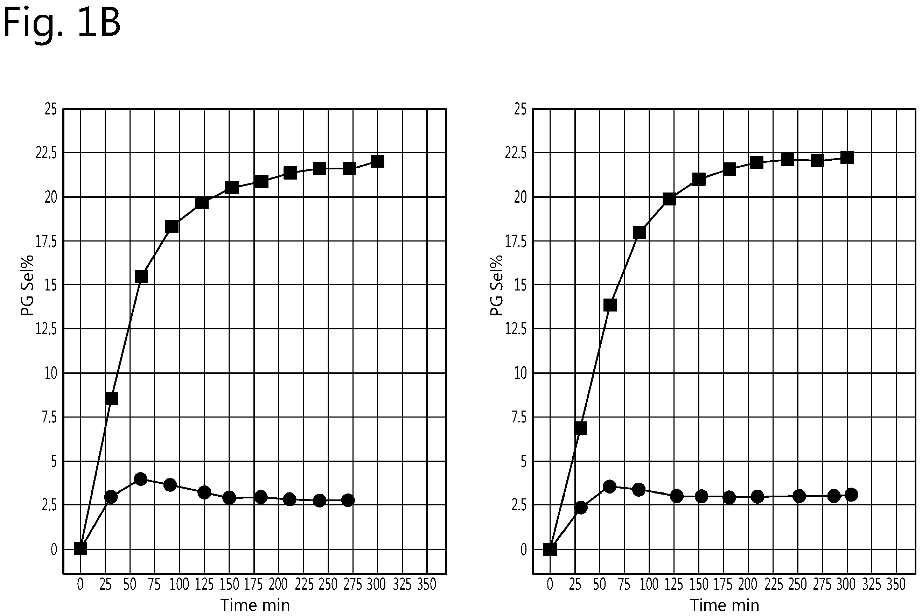

Figure 1B: selectivity of propylene glycol obtained in the product stream, for sucrose as feed (squares) and glucose as feed (circles) for a residence time of about 24 minutes (left hand) and for a residence time of about 34 minutes (right hand).

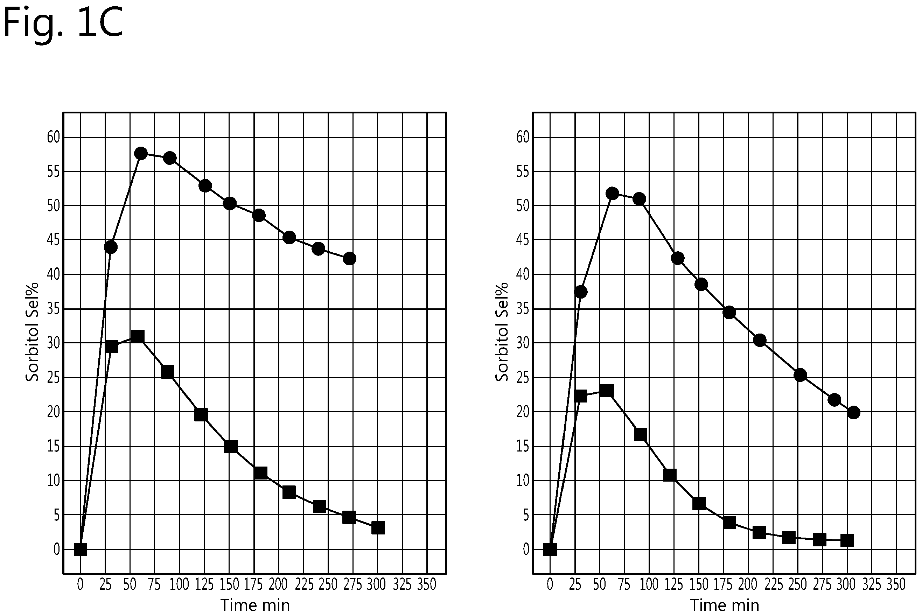

Figure 1C: selectivity of sorbitol obtained in the product stream, for sucrose as feed (squares) and glucose as feed (circles) for a residence time of about 24 minutes (left hand) and for a residence time of about 34 minutes (right hand).

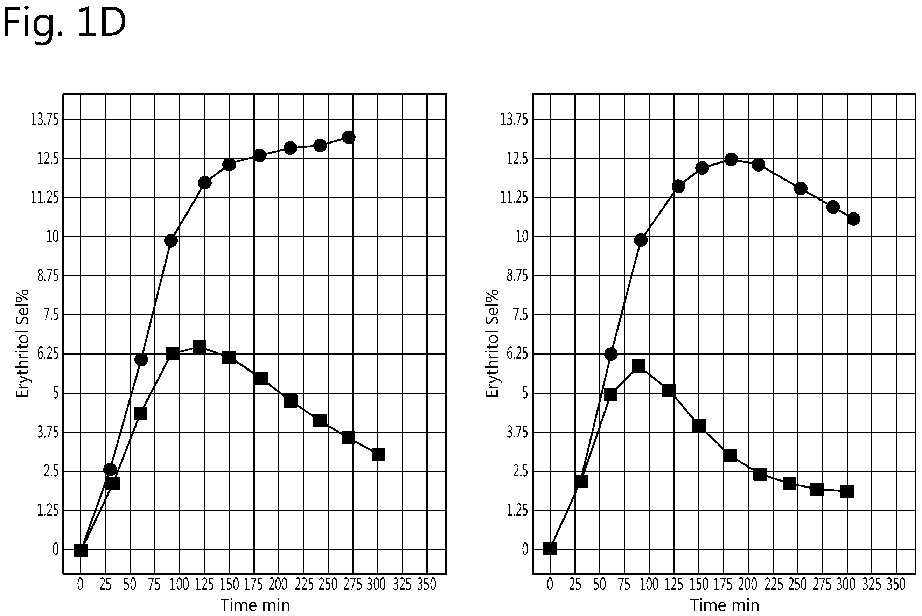

Figure 1D: selectivity of erythritol obtained in the product stream, for sucrose as feed (squares) and glucose as feed (circles) for a residence time of about 24 minutes (left hand) and for a residence time of about 34 minutes (right hand).

From the figures it can be concluded that when using sucrose as a feed much lower selectivities are obtained for both sorbitol and erythritol as opposed to the combined selectivity for ethylene glycol and propylene glycol, when compared to the selectivities for glucose as a feed.

## Claims

1. A process for producing a mixture of 2 to 40% by weight of glycols dissolved in water with a ratio of between 1 : 5 to 1 : 100 for the combined selectivity for erythritol and sorbitol : the combined selectivity for ethylene glycol and propylene glycol, of the reaction products produced by said process, which process comprises feeding to a pressurized, continuously stirred tank reactor hydrogen and an aqueous feed solution comprising water and a carbohydrate, wherein the reactor contains a catalyst system which comprises a homogeneous catalyst comprising a tungsten compound and a heterogeneous catalyst comprising a hydrogenolysis metal selected from the groups 8, 9 or 10 of the Periodic Table of the Elements, **characterized in that** the carbohydrate in the aqueous feed comprises at least 80% of sucrose, by weight based on the total amount of carbohydrate in the aqueous feed.

2. A process according to claim 1, wherein the ratio for the combined selectivity for erythritol and sorbitol : the combined selectivity for ethylene glycol and propylene glycol of the reaction products produced by said process is at least 1 : 7, preferably at least 1 : 10, more preferably at least 1 : 15.

3. A process according to any of the preceding claims, wherein the carbohydrate in the aqueous feed comprises at least 90% of sucrose, preferably at least 95% by weight of sucrose, by weight based on the total amount of carbohydrate in the aqueous feed.

4. A process according to any of the preceding claims, wherein the aqueous feed solution comprising water and a carbohydrate comprises between 5 and 35% by weight of carbohydrate, preferably between 10 and 30% by weight of carbohydrate.

5. A process according to any of the preceding claims, wherein the hydrogenolysis metal from groups 8, 9 or 10 of the Periodic Table of the Elements is selected from the group consisting of Cu, Fe, Ni, Co, Pd, Pt, Ru, Rh, Ir, Os and combinations thereof, and preferably such is ruthenium.

6. A process according to any of the preceding claims, wherein the amount of the hydrogenolysis metal selected from the groups 8, 9 or 10 of the Periodic Table of the Elements present in the reactors is between 0.05 and 20 g hydrogenolysis metal / L of reactor volume , preferably between 0.1 and 12 g g hydrogenolysis metal / L of reactor volume, more preferably between 0.5 and 8 g hydrogenolysis metal / L of reactor volume.

7. A process according to any of the preceding claims, wherein the hydrogenolysis metal from groups 8, 9 or 10 of the Periodic Table of the Elements is present in the form of a catalyst supported on a carrier, wherein the carriers preferably is selected from the group supports, consisting of activated carbon, silica, alumina, silica-alumina, zirconia, titania, niobia, iron oxide, tin oxide, zinc oxide, silica-zirconia, zeolites, aluminosilicates, titanosilicates, magnesia, silicon carbide, clays and combinations thereof.

8. A process according to any of the preceding claims, wherein the homogeneous catalyst comprising a tungsten compound is selected from the group consisting of tungstic acid (H₂WO₄), ammonium tungstate, ammonium metatungstate, ammonium paratungstate, tungstate compounds comprising at least one Group 1 or 2 element, metatungstate compounds comprising at least one Group 1 or 2 element, paratungstate compounds comprising at least one Group 1 or 2 element, tungsten oxide (WO₃), heteropoly compounds of tungsten, and combinations thereof.

9. A process according to any of the preceding claims, wherein the homogeneous catalyst comprising a tungsten compound is dissolved or dispersed in water and/or an alkylene glycol.

10. A process according to any of the preceding claims, wherein the homogeneous catalyst comprising a tungsten compound is continuously or periodically added to the reactor.

11. A process according to any of the preceding claims, wherein the amount of homogeneous catalyst present in the reactor is between 0.05 and 5 wt.%, preferably between 0.1 and 2 wt.%, calculated as tungsten metal.

12. A process according to any of the preceding claims, wherein the homogeneous catalyst comprising a tungsten compound and a heterogeneous catalyst comprising a hydrogenolysis metal selected from the groups 8, 9 or 10 of the Periodic Table of the Elements are present in the reactor in amounts such that the weight ratio of weight of tungsten to the total weight of hydrogenolysis metal, all calculated on metal basis, is between 1: 3000 to 50 : 1 (tungsten metal : transition metal wt : wt).

13. A process according to any of the preceding claims, wherein the reactor is pressurized with hydrogen.

14. A process for producing glycols dissolved in water, which process comprises feeding to a pressurized, continuously stirred tank reactor hydrogen an aqueous feed solution comprising water and a carbohydrate, wherein the reactor comprises a catalyst system which comprises a homogeneous catalyst comprising a tungstic acid and a heterogeneous catalyst comprising ruthenium supported on a carrier, **characterized in that** the carbohydrate in the aqueous feed comprises at least 80% of sucrose, by weight based on the total amount of carbohydrate in the aqueous feed.

15. A process according to any of the preceding claims, wherein the aqueous feed solution comprising water and a carbohydrate comprises between 40% and 85% of water, between 5 and 35% by weight of carbohydrate, and between 5 to 40% of an alkylene glycol co-solvent, all by weight on the total aqueous feed solution.

## Patentansprüche

1. Verfahren zum Herstellen einer Mischung aus 2 bis 40 Gew.-% Glykolen, die in Wasser gelöst sind, mit einem Verhältnis zwischen 1 : 5 bis 1 : 100 für die kombinierte Selektivität für Erythrit und Sorbit : die kombinierte Selektivität für Ethylenglykol und Propylenglykol, der Reaktionsprodukte, die durch das Verfahren hergestellt werden, wobei das Verfahren ein Zuführen zu einem unter Druck stehenden Rührkesselreaktor von Wasserstoff und einer wässrigen Suspensionslösung umfasst, umfassend Wasser und ein Kohlenhydrat, wobei der Reaktor ein Katalysatorsystem enthält, das einen homogenen Katalysator, umfassend eine Wolframverbindung, und einen heterogenen Katalysator umfasst, umfassend ein Hydrogenolysemetall, das aus den Gruppen 8, 9 oder 10 des Periodensystems der Elemente ausgewählt ist,
**dadurch gekennzeichnet, dass** das Kohlenhydrat in der wässrigen Suspension mindestens 80 Gew.-% Saccharose umfasst, basierend auf der Gesamtmenge von Kohlenhydrat in der wässrigen Suspension.

2. Verfahren nach Anspruch 1, wobei das Verhältnis für die kombinierte Selektivität für Erythrit und Sorbit : die kombinierte Selektivität für Ethylenglykol und Propylenglykol der Reaktionsprodukte, die durch das Verfahren hergestellt werden, mindestens 1 : 7, vorzugsweise mindestens 1 : 10, stärker bevorzugt mindestens 1 : 15, beträgt.

3. Verfahren nach einem der vorstehenden Ansprüche, wobei das Kohlenhydrat in der wässrigen Suspension mindestens 90 Gew.-% Saccharose, vorzugsweise mindestens 95 Gew.-% Saccharose umfasst, basierend auf der Gesamtmenge von Kohlenhydrat in der wässrigen Suspension.

4. Verfahren nach einem der vorstehenden Ansprüche, wobei die wässrige Suspensionslösung, umfassend Wasser und ein Kohlenhydrat, zwischen 5 und 35 Gew.-% Kohlenhydrat, vorzugsweise zwischen 10 und 30 Gew.-% Kohlenhydrat, umfasst.

5. Verfahren nach einem der vorstehenden Ansprüche, wobei das Hydrogenolysemetall aus den Gruppen 8, 9 oder 10 des Periodensystems der Elemente aus der Gruppe ausgewählt ist, bestehend aus Cu, Fe, Ni, Co, Pd, Pt, Ru, Rh, Ir, Os und Kombinationen davon, und vorzugsweise Ruthenium ist.

6. Verfahren nach einem der vorstehenden Ansprüche, wobei die Menge des Hydrogenolysemetalls, das aus den Gruppen 8, 9 oder 10 des Periodensystems der Elemente ausgewählt ist, in den Reaktoren zwischen 0,05 und 20 g Hydrogenolysemetall/L Reaktorvolumen, vorzugsweise zwischen 0,1 und 12 g g Hydrogenolysemetall/L Reaktorvolumen, stärker bevorzugt zwischen 0,5 und 8 g Hydrogenolysemetall/L Reaktorvolumen, vorhanden ist.

7. Verfahren nach einem der vorstehenden Ansprüche, wobei das Hydrogenolysemetall aus den Gruppen 8, 9 oder 10 des Periodensystems der Elemente in der Form eines Katalysators vorhanden ist, der auf einem Träger getragen wird, wobei die Träger vorzugsweise aus den Gruppenträgern ausgewählt sind, bestehend aus Aktivkohle, Siliziumoxid, Aluminiumoxid, Siliziumoxid-Aluminiumoxid, Zirkonoxid, Titanoxid, Niob, Eisenoxid, Zinnoxid, Zinkoxid, Siliziumoxid-Zirkonoxid, Zeolithen, Aluminosilikaten, Titanosilikaten, Magnesiumoxid, Siliziumkarbid, Tonen und Kombinationen davon.

8. Verfahren nach einem der vorstehenden Ansprüche, wobei der homogene Katalysator, umfassend eine Wolframverbindung, aus der Gruppe ausgewählt ist, bestehend aus Wolframsäure (H₂WO₄), Ammoniumwolframat, Ammoniummetawolframat, Ammoniumparawolframat, Wolframatverbindungen, umfassend mindestens ein Gruppe 1 oder 2-Element, Metawolframatverbindungen, umfassend mindestens ein Gruppe 1 oder 2-Element, Parawolframatverbindungen, umfassend mindestens ein Gruppe 1 oder 2-Element, Wolframoxid (WOs), Heteropolyverbindungen von Wolfram und Kombinationen davon.

9. Verfahren nach einem der vorstehenden Ansprüche, wobei der homogene Katalysator, umfassend eine Wolframverbindung, in Wasser und/oder einem Alkylenglykol gelöst oder dispergiert ist.

10. Verfahren nach einem der vorstehenden Ansprüche, wobei der homogene Katalysator, umfassend eine Wolframverbindung, kontinuierlich oder periodisch dem Reaktor zugesetzt wird.

11. Verfahren nach einem der vorstehenden Ansprüche, wobei die Menge des homogenen Katalysators, der in dem Reaktor vorhanden ist, zwischen 0,05 und 5 Gew.-%, vorzugsweise zwischen 0,1 und 2 Gew.-% beträgt, berechnet als Wolframmetall.

12. Verfahren nach einem der vorstehenden Ansprüche, wobei der homogene Katalysator, umfassend eine Wolframverbindung, und ein heterogener Katalysator, umfassend ein Hydrogenolysemetall, das aus den Gruppen 8, 9 oder 10 des Periodensystems der Elemente ausgewählt ist, in dem Reaktor in derartigen Mengen vorhanden sind, dass das Gewichtsverhältnis von Gewicht von Wolfram zu dem Gesamtgewicht von Hydrogenolysemetall, alle auf Metallbasis berechnet, zwischen 1 : 3000 bis 50 : 1 (Wolframmetall : Übergangsmetall Gew.-% : Gew.-%) beträgt.

13. Verfahren nach einem der vorstehenden Ansprüche, wobei der Reaktor mit Wasserstoff unter Druck gesetzt ist.

14. Verfahren zum Herstellen von Glykolen, die in Wasser gelöst sind, wobei das Verfahren das Zuführen zu einem unter Druck stehenden Rührkesselreaktor von Wasserstoff einer wässrigen Suspensionslösung umfasst, umfassend Wasser und ein Kohlenhydrat, wobei der Reaktor ein Katalysatorsystem umfasst, das einen homogenen Katalysator, umfassend eine Wolframsäure, und einen heterogenen Katalysator umfasst, umfassend Ruthenium, getragen auf einem Träger, **dadurch gekennzeichnet, dass** das Kohlenhydrat in der wässrigen Suspension mindestens zu 80 Gew.-% Saccharose umfasst, basierend auf der Gesamtmenge von Kohlenhydrat in der wässrigen Suspension.

15. Verfahren nach einem der vorstehenden Ansprüche, wobei die wässrige Suspensionslösung, umfassend Wasser und ein Kohlenhydrat, zwischen 40 Gew.-% und 85 Gew.-% Wasser, zwischen 5 und 35 Gew.-% Kohlenhydrat und zwischen 5 bis 40 Gew.-% ein Alkylenglykolcolösungsmittel umfasst, alle nach Gewicht in der gesamten wässrigen Suspensionslösung.

## Revendications

1. Procédé de production d'un mélange de 2 à 40 % en poids de glycols dissous dans l'eau avec un rapport compris entre 1: 5 et 1: 100 pour la sélectivité combinée pour l'érythritol et le sorbitol : la sélectivité combinée pour l'éthylène glycol et le propylène glycol, des produits de réaction produits par ledit procédé, lequel procédé comprend l'introduction dans un réacteur à cuve sous pression, à agitation continue, d'hydrogène et d'une solution de charge aqueuse comprenant de l'eau et un glucide, dans lequel le réacteur contient un système de catalyseur qui comprend un catalyseur homogène comprenant un composé de tungstène et un catalyseur hétérogène comprenant un métal d'hydrogénolyse choisi parmi les groupes 8, 9 ou 10 du tableau périodique des éléments, **caractérisé en ce que** le glucide dans la charge aqueuse comprend au moins 80 % de saccharose, en poids sur la base de la quantité totale de glucide dans la charge aqueuse.

2. Procédé selon la revendication 1, dans lequel le rapport pour la sélectivité combinée pour l'érythritol et le sorbitol : la sélectivité combinée pour l'éthylène glycol et le propylène glycol des produits de réaction produits par ledit procédé est d'au moins 1: 7, de préférence au moins 1: 10, plus préférablement au moins 1: 15.

3. Procédé selon l'une quelconque des revendications précédentes, dans lequel le glucide dans la charge aqueuse comprend au moins 90 % de saccharose, de préférence au moins 95 % en poids de saccharose, en poids sur la base de la quantité totale de glucide dans la charge aqueuse.

4. Procédé selon l'une quelconque des revendications précédentes, dans lequel la solution de charge aqueuse comprenant de l'eau et un glucide comprend entre 5 et 35 % en poids de glucide, de préférence entre 10 et 30 % en poids de glucide.

5. Procédé selon l'une quelconque des revendications précédentes, dans lequel le métal d'hydrogénolyse des groupes 8, 9 ou 10 du tableau périodique des éléments est choisi dans le groupe constitué de Cu, Fe, Ni, Co, Pd, Pt, Ru, Rh, Ir, Os et combinaisons de ceux-ci, et de préférence il s'agit du ruthénium.

6. Procédé selon l'une quelconque des revendications précédentes, dans lequel la quantité du métal d'hydrogénolyse choisi parmi les groupes 8, 9 ou 10 du tableau périodique des éléments présent dans les réacteurs est comprise entre 0,05 et 20 g de métal d'hydrogénolyse/L de volume de réacteur, de préférence entre 0,1 et 12 g g de métal d'hydrogénolyse/L de volume de réacteur, plus préférablement entre 0,5 et 8 g de métal d'hydrogénolyse/L de volume de réacteur.

7. Procédé selon l'une quelconque des revendications précédentes, dans lequel le métal d'hydrogénolyse des groupes 8, 9 ou 10 du tableau périodique des éléments est présent sous la forme d'un catalyseur supporté sur un support, dans lequel les supports sont de préférence choisis dans le groupe supports, constitué de charbon actif, silice, alumine, silice-alumine, zircone, dioxyde de titane, oxyde de niobium, oxyde de fer, oxyde d'étain, oxyde de zinc, silice-zircone, zéolithes, aluminosilicates, titanosilicates, magnésie, carbure de silicium, argiles et combinaisons de ceux-ci.

8. Procédé selon l'une quelconque des revendications précédentes, dans lequel le catalyseur homogène comprenant un composé de tungstène est choisi dans le groupe constitué d'acide tungstique (H₂WO₄), tungstate d'ammonium, métatungstate d'ammonium, paratungstate d'ammonium, composés de tungstate comprenant au moins un élément du groupe 1 ou 2, composés de métatungstate comprenant au moins un élément du groupe 1 ou 2, composés de paratungstate comprenant au moins un élément du groupe 1 ou 2, oxyde de tungstène (WOs), hétéropolycomposés de tungstène, et combinaisons de ceux-ci.

9. Procédé selon l'une quelconque des revendications précédentes, dans lequel le catalyseur homogène comprenant un composé de tungstène est dissous ou dispersé dans de l'eau et/ou un alkylène glycol.

10. Procédé selon l'une quelconque des revendications précédentes, dans lequel le catalyseur homogène comprenant un composé de tungstène est ajouté de manière continue ou périodique au réacteur.

11. Procédé selon l'une quelconque des revendications précédentes, dans lequel la quantité de catalyseur homogène présent dans le réacteur est comprise entre 0,05 et 5 % en poids, de préférence entre 0,1 et 2 % en poids, calculée en tant que tungstène métallique.

12. Procédé selon l'une quelconque des revendications précédentes, dans lequel le catalyseur homogène comprenant un composé de tungstène et un catalyseur hétérogène comprenant un métal d'hydrogénolyse choisi parmi les groupes 8, 9 ou 10 du tableau périodique des éléments sont présents dans le réacteur en des quantités telles que le rapport en poids du poids de tungstène au poids total de métal d'hydrogénolyse, tous calculés sur base du métal, est compris entre 1: 3 000 et 50: 1 (tungstène métallique : métal de transition poids : poids).

13. Procédé selon l'une quelconque des revendications précédentes, dans lequel le réacteur est mis sous pression avec de l'hydrogène.

14. Procédé de production de glycols dissous dans de l'eau, lequel procédé comprend l'introduction dans un réacteur à cuve sous pression, à agitation continue, d'hydrogène et d'une solution de charge aqueuse comprenant de l'eau et un glucide, dans lequel le réacteur comprend un système catalytique qui comprend un catalyseur homogène comprenant un acide tungstique et un catalyseur hétérogène comprenant du ruthénium supporté sur un support, **caractérisé en ce que** le glucide dans la charge aqueuse comprend au moins 80 % de saccharose, en poids sur la base de la quantité totale de glucide dans la charge aqueuse.

15. Procédé selon l'une quelconque des revendications précédentes, dans lequel la solution de charge aqueuse comprenant de l'eau et un glucide comprend entre 40 % et 85 % d'eau, entre 5 et 35 % en poids de glucide, et entre 5 et 40 % d'un co-solvant alkylène glycol, tous en poids de la solution de charge aqueuse totale.
